# FASCICULE DE BREVET EUROPEEN

(11) **EP 0 606 441 B1**
(45) Date de publication et mention de la délivrance du brevet: **11.11.1998**
(21) Numéro de dépôt: 93916024.8
(22) Date de dépôt: 21.07.1993
(51) Int. Cl.: C12P 7/24, C12P 17/04

(54) **PROCEDE DE PREPARATION DE SUBSTANCES AROMATIQUES PAR VOIE ENZYMATIQUE**
VERFAHREN ZUR ENZYMATISCHEN HERSTELLUNG VON AROMATISCHEN SUBSTANZEN
METHOD FOR ENZYMATICALLY PREPARING AROMATIC SUBSTANCES

(30) Priorité: 24.07.1992 FR 9209174
(43) Date de publication de la demande: 20.07.1994
(73) Titulaire: ETABLISSEMENTS V. MANE FILS, 06620 Le-Bar-sur-Loup (FR)
(72) Inventeur: MANE, Jean, F-06130 Grasse (FR); ZUCCA, Joseph, "Les Genêts", F-06600 Antibes (FR)
(74) Mandataire: Boulinguiez, Didier
(86) Numéro de dépôt international: FR9300745
(87) Numéro de publication internationale: WO9402621

(56) Documents cités:
- EP-A- 0 233 570
- EP-A- 0 453 368
- EP-A- 0 542 348
- US-A- 5 128 253
- CHEMICAL ABSTRACTS, vol. 106, no. 25, 22 Juin 1987, Columbus, Ohio, US; abstract no. 212647y, MTEBE, KASWIJA ET AL. 'Volatiles derived from lipoxygenase-catalyzed reactions in winged beans (Psophocarpus tetragonolobus).' page 547 ;
- CHEMICAL ABSTRACTS, vol. 114, no. 3, 21 Janvier 1991, Columbus, Ohio, US; abstract no. 22487p, YOSHIMOTO,TOMOTAKA ET AL. 'Manufacture of benzaldehydes from styrenes with dioxygenase from Pseudomonas species.' page 575 ;
- JOURNAL OF FOOD SCIENCE vol. 50, no. 1, Février 1985, CHICAGO US pages 5 - 9 DAVID B. JOSEPHSON ET AL. 'Volatile compounds characterizing the aroma of fresh atlantic and pacific Oysters.'
- OCL 2, 5, 1995, 374 - 385 D. Martini et G. Iacazio "Les lipoxygénases et les voies métaboliques associées"
- Traduction en langue française du JP-A-2 200 192

## Description

### Description pour les Etats contractants suivants : AT, BE, CH, DE, DK, ES, FR, GB, IE, GR, IT, LI, NL, PT, SE

L'invention a pour objet un procédé de préparation de certaines substances aromatiques par voie enzymatique.

Par substances aromatiques on entend toutes substances utilisées pour le parfum qu'elles dégagent et/ou le goût qu'elles procurent dans l'industrie alimentaire, pharmaceutique ou de la parfumerie.

Le procédé conforme à l'invention permet la préparation de substances aromatiques de formule : 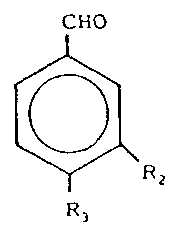 dans laquelle
R₂ peut être un radical -H, -OH, -O-CH₃,
R₃ peut être un radical -H, -OH, -O-CH₃,
R₂ et R₃ peuvent former un pont méthylènedioxy

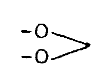

Avantageusement ces substances aromatiques appartiennent au groupe comprenant : la vanilline, le benzaldéhyde, l'aldéhyde anisique, l'aldéhyde para-hydroxybenzoïque, l'héliotropine et l'aldéhyde protocatéchique.

Jusqu'à présent, il était connu de préparer la vanilline selon différents procédés :
- à partir de guaïacol, par voie chimique, mais la vanilline est obtenue dans un mélange comprenant de nombreuses impuretés, d'où des rendements moyens ;
- à partir de la dégradation de la lignine, mais l'obtention de la vanilline à l'état pur nécessite une grande consommation de produits chimiques et d'énergie et occasionne de nombreux rejets écotoxiques ;
- à partir de safrole, composé présent dans l'essence de sassafras permettant ainsi l'obtention d'un mélange isovanilline-vanilline qui sont deux isomères difficiles à séparer ;
- à partir d'eugénol, composé présent dans l'huile de clou de girofle, ce composé subissant une première étape d'isomérisation en isoeugénol (cis et trans en proportions variables) puis une deuxième étape d'oxydation pour donner la vanilline ; cependant, ce procédé chimique donne des rendements très moyens.

De plus, tous les procédés de l'art antérieur faisant appel à au moins une manipulation chimique, le produit obtenu ne peut qu'être appelé "vanilline de synthèse ou d'hémisynthèse" et ne peut en aucun cas être appelé "vanilline d'origine naturelle", même si le substrat de départ est naturel (cas de l'eugénol ou du safrole).

En ce qui concerne le benzaldéhyde, il est produit par exemple à partir d'aldéhyde cinnamique traité en milieu alcalin sous pression et à haute température (brevet US 4 617 419).

Quant à l'aldéhyde anisique, il est classiquement produit par oxydation chimique de l'anéthole que l'on trouve en grande quantité dans l'essence de badiane.

Mais, tout comme la vanilline et pour les mêmes raisons, ces deux composés ne peuvent pas être qualifiés de "naturels".

JP-A-2 200 192 décrit un procédé naturel de préparation d'aldéhydes consistant à mettre en contact une enzyme produite par une souche appartenant au genre Pseudomonas et capable de produire une enzyme à activité dioxygénase avec des dérivés styrèniques ayant une insaturation située exclusivement en a du noyau benzénique. L'activité de cette enzyme est faible. La nature pathogène-opportuniste du microorganisme qui la produit, constitue un obstacle à l'utilisation des aldéhydes obtenus dans des produits alimentaires, pharmaceutiques ou cosmétiques.

L'invention a pour but de remédier aux inconvénients de l'art antérieur et propose un procédé biotechnologique permettant la préparation de certaines substances aromatiques de bonne pureté avec des rendements élevés.

EP-A-0542348 (QUEST), document de l'art antérieur selon Article 54(3)CBE, décrit un procédé biotechnologique de préparation de substances aromatiques telles que la vanilline. Dans ce procédé, on soumet à l'action d'une lipoxygénase des substrats ayant la formule suivante : 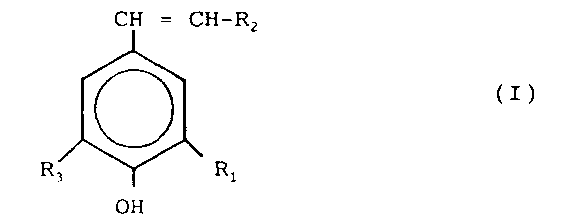 dans laquelle :
- R₁ et R₃ représentent indépendamment H, -OH, -OCH₃, alkyle en C₁-C₄,
- R₂ représente H, -CH₂OH, -COOH, -OCH₃, alkyle en C₁-C₆,
- CH₂O-R₄ avec R₄ = benzoyle ou alkanoyle, ou
- R₂ est un radical -CO-CH₂-CO- reliant deux fractions moléculaires de formule (I) ci-dessus.

Les substrats décrits dans ce brevet sont exclus de la présente invention.

La Société Demanderesse a eu le mérite d'établir qu'il était possible de préparer des substances aromatiques de formule : 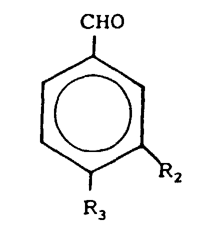 en soumettant l'un de leurs précurseurs, appelé substrat, à l'action d'une enzyme : la lipoxygénase ou lipoxidase, ledit substrat étant choisi parmi l'estragol, le safrole et les composés répondant à la formule: 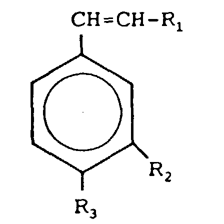 dans laquelle :
R₁ peut être un radical -CH₃, -CH₂OH, -CHO, -COOH, 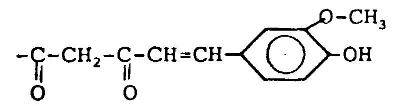
R₂ peut être un radical -H, -OH, -O-CH₃,
R₃ peut être un radical -H, -OH, -O-CH₃,
R₂ et R₃ peuvent former un pont méthylènedioxy 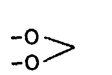 à la condition que lorsque R₁ représente -CH₃, CH₂OH ou -COOH, alors R₃ ne représente pas -OH et que lorsque R₁ représente 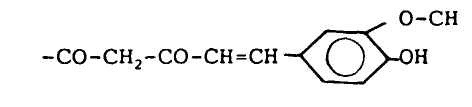 et R₂ représente -OCH₃ alors R₃ ne représente pas -OH.

L'enzyme utilisée est répertoriée sous la référence EC 1 13 11 12 dans l'ouvrage "Enzyme Nomenclature" (IU BMB Academic Press 1992).

Elle est naturellement présente dans de nombreux végétaux tels que par exemple le soja, le blé ou la betterave, dans certains organes d'animaux tels que par exemple les foies de porc, de veau ou de poisson. Ces matières végétales ou animales peuvent donc constituer des sources industrielles de cette enzyme qui, de plus, peut également être synthétisée par certains microorganismes.

La lipoxygénase est connue notamment pour sa participation à la première étape d'un procédé permettant la synthèse d'aldéhydes à partir d'acides gras. En effet, son activité catalytique oxydative sur les acides gras insaturés, et plus particulièrement sur les acides linoléique et linolénique, donne lieu à la formation d'un hydroperoxyde pouvant ensuite être scindé en un oxoacide et un aldéhyde par une hydroperoxyde lyase.

Or, dans le cadre de l'invention, il a été trouvé que la lipoxygénase, quelle que soit son origine, pouvait à elle seule permettre la préparation de substances aromatiques de formule : 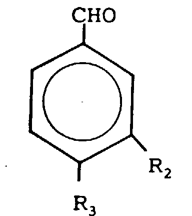 à partir de substrats choisis parmi l'estragol, le safrole et les composés représentés par la formule ci-dessus mentionnée et selon les réactions suivantes : 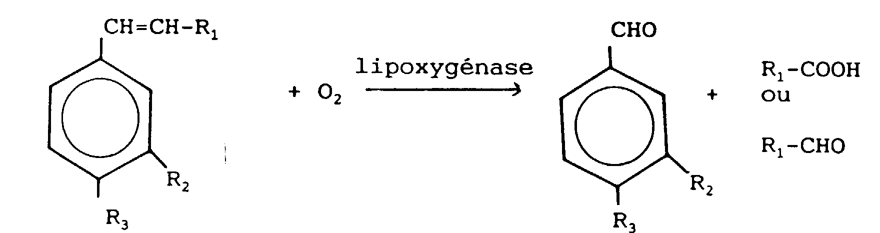 (R₁, R₂ et R₃ ayant les significations données précédemment),

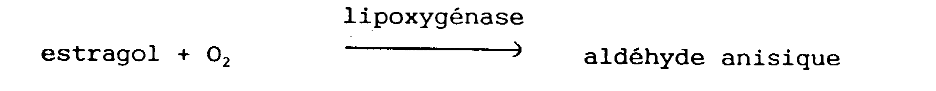

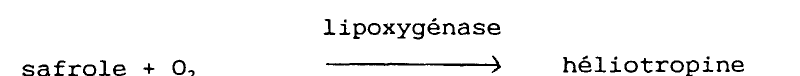

Les substances aromatiques préférées préparées selon le procédé conforme à l'invention sont récapitulées dans le tableau I.

**TABLEAU I**

| R₁ | R₂ | R₃ | SUBSTRAT | ISOMERE | PRODUIT |
|---|---|---|---|---|---|
| -CHO | -O-CH₃ | -OH | aldéhyde coniférylique | | vanilline |
| -CH₂OH | -H | -H | alcool cinnamique | | benzaldéhyde |
| -CHO | -H | -H | aldéhyde cinnamique | | benzaldéhyde |
| -COOH | -H | -H | acide cinnamique | | benzaldéhyde |
| -CH₃ | -H | -O-CH₃ | anéthol | estragol | aldéhyde anisique |
| -CH₃ | -O O- | | isosafrole | safrole | héliotropine |
| -COOH | -OH | -OH | acide caféique | | aldéhyde proto catéchique |

L'estragol et le safrole qui sont respectivement les isomères de l'anéthole et de l'isosafrole peuvent eux-mêmes être transformés par la lipoxygénase en l'aldéhyde correspondant, soit l'aldéhyde anisique et l'héliotropine mais avec un rendement plus faible.

En effet la condition la plus importante pour que la lipoxygénase puisse exercer son action est que le substrat comporte une liaison insaturée correctement positionnée : c'est le cas de toutes les molécules représentées par la formule répondant au "substrat" dont la liaison insaturée située sur la chaîne portant R₁ ou CH₂ est positionnée soit en alpha, soit en bêta du cycle aromatique. C'est aussi le cas des exemples connus que sont les acides linoléique et linolénique qui voient soit leur carbone 9, soit leur carbone 13 (constituant un système cis-cis diénique non conjugué) être le siège d'une peroxydation.

Le procédé conforme à l'invention consiste donc à mettre en présence, en milieu tamponné, agité et thermo-régulé, une lipoxygénase et un substrat choisi parmi ceux mentionnés ci-dessus pendant un temps suffisant pour permettre la préparation de la substance aromatique correspondante, celle-ci pouvant être ensuite récupérée par des techniques classiques d'extraction par solvant ou par distillation.

Dans le procédé conforme à l'invention, la lipoxygénase peut être utilisée sous forme purifiée ou non purifiée.

Avantageusement,
- lorsqu'elle est purifiée, c'est-à-dire généralement quand elle présente une activité supérieure à 30000 u/mg, (1 unité correspondant à une augmentation de l'absorbance à 234 nm d'une valeur de 0,001 par minute lorsque l'acide linoléique est le substrat, à pH 9 et à 25°C), la lipoxygénase est utilisée à raison de 1 à 500 mg par gramme de substrat, de préférence de 5 à 100 mg par gramme de substrat et plus préférentiellement encore de 10 à 50 mg par gramme de substrat, (à titre d'exemple, on peut utiliser une lipoxygénase commercialisée par SIGMA sous forme d'une préparation enzymatique contenant au moins 60% de protéines), et
- lorsqu'elle n'est pas purifiée, c'est-à-dire lorsqu'elle contient moins de 7500 u/mg, comme c'est le cas dans les poudres acétoniques du commerce (forme préconcentrée à partir d'une matière première), ou dans un végétal frais broyé ou dans une poudre de farine végétale, ou encore, dans un organe animal frais broyé tel que le foie, elle sera utilisée à raison de 0,01 à 50g par gramme de substrat, de préférence de 0,025 à 10 g par gramme de substrat et plus préférentiellement encore, de 0,1 à 5 g par gramme de substrat.

Selon le procédé conforme à l'invention, les lipoxygénases préférentiellement utilisées sont la lipoxygénase de germes de soja et la lipoxygénase de germes de blé.

Selon un mode de réalisation avantageux du procédé conforme à l'invention, on établit la concentration en substrat de la lipoxygénase entre 2,5 et 100 g par litre de milieu de réaction et de préférence entre 20 et 75 g par litre de milieu de réaction.

Conformément à l'invention, ce milieu de réaction est un milieu aqueux tamponné. Les tampons utilisés sont des tampons classiques tels que le tampon phosphate de potassium et/ou le tampon phosphate de sodium et/ou le tampon borate de sodium et/ou le tampon phosphate-citrate-borate de sodium.

Avantageusement, les molarités de ces tampons sont comprises entre 0,05 M et 0,5 M et leur pH est compris entre 2 et 10 et de préférence entre 5 et 10. Les ajustements de pH se font soit avec de la potasse, soit avec de la soude, soit avec de l'acide phosphorique.

Selon d'autres caractéristiques avantageuses de l'invention :
- la température du milieu de réaction varie entre 4 et 40°C et de préférence entre 22 et 32°C ;
- la durée de la réaction varie entre 30 minutes et 7 jours, de préférence entre 2 heures et 5 jours et est déterminée par suivi cinétique des réactions ;
- l'agitation doit être suffisamment forte, pour homogénéiser ou émulsionner l'ensemble du milieu réactionnel et pour oxygéner tout le substrat présent, et est réalisée à une vitesse comprise entre 150 et 250 tr/min.

En outre, un agent émulsifiant tel que le TWEEN 80® peut être introduit dans le milieu à raison de 0,05 à 2% en volume par rapport au milieu de réaction.

Selon un mode de réalisation avantageux du procédé conforme à l'invention, en fonction du substrat utilisé, et en particulier quand le substrat est un composé de nature phénolique, du fait de la toxicité des phénols vis-à-vis de l'enzyme, il peut être nécessaire d'ajouter dans le milieu de réaction au moins un adsorbant ou un complexant choisi dans le groupe comprenant le charbon actif à une concentration comprise entre 5 et 50 g par litre, celui connu sous la marque Amberlite de type XAD₂ à une concentration comprise entre 5 et 50 g par litre, la bêta cyclodextrine à une concentration comprise entre 3 et 18 g par litre et la polyvinyl polypyrrolidone (PVPP) à une concentration comprise entre 10 et 60 g par litre, de façon à protéger ladite enzyme.

L'invention ne se limite pas strictement au procédé décrit, elle en embrasse au contraire toutes les variantes. La Société Demanderesse a par exemple expérimenté de façon concluante la transformation du bêta-carotène en béta-ionone par l'action de la lipoxygénase qui agit dans ce cas au niveau d'une liaison insaturée située en position gamma par rapport au cycle de la molécule, conjuguée avec une liaison insaturée située en position alpha par rapport au même cycle.

Les exemples qui suivent permettront de mieux comprendre l'invention ; ils ne sont pas limitatifs et concernent les modes de réalisation avantageux de l'invention.

### EXEMPLE 1

Dans un flacon contenant 20 ml de tampon borate de sodium présentant une molarité de 0,1M et un pH de 6,5, on ajoute 25 g/l d'aldéhyde cinnamique, 20 mg de lipoxygénase issue de germes de soja et titrant 36000 u/mg et 0,05 g de TWEEN 80®.

Après quatre jours d'incubation à 28°C et sous agitation à une vitesse de 150 tr/min, on détecte 1,1 g/l de benzaldéhyde dans le milieu.

### EXEMPLE 2

Dans les mêmes conditions que dans l'exemple 1, on ajoute 8 g/l d'anéthole.

Après quatre jours, on mesure 448 mg/l d'aldéhyde anisique.

### Description pour les Etats contractants suivants : LU, MC

L'invention a pour objet un procédé de préparation de certaines substances aromatiques par voie enzymatique.

Par substances aromatiques on entend toutes substances utilisées pour le parfum qu'elles dégagent et/ou le goût qu'elles procurent dans l'industrie alimentaire, pharmaceutique ou de la parfumerie.

Le procédé conforme à l'invention permet la préparation de substances aromatiques de formule : 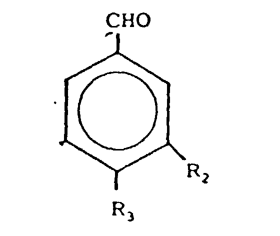 dans laquelle
R₂ peut être un radical -H, -OH, -O-CH₃,
R₃ peut être un radical -H, -OH, -O-CH₃,
R₂ et R₃ peuvent former un pont méthylènedioxy

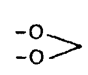

Avantageusement ces substances aromatiques appartiennent au groupe comprenant : la vanilline, le benzaldéhyde, l'aldéhyde anisique, l'aldéhyde para-hydroxybenzoïque, l'héliotropine et l'aldéhyde protocatéchique.

Jusqu'à présent, il était connu de préparer la vanilline selon différents procédés :
- à partir de guaïacol, par voie chimique, mais la vanilline est obtenue dans un mélange comprenant de nombreuses impuretés, d'où des rendements moyens ;
- à partir de la dégradation de la lignine, mais l'obtention de la vanilline à l'état pur nécessite une grande consommation de produits chimiques et d'énergie et occasionne de nombreux rejets écotoxiques ;
- à partir de safrole, composé présent dans l'essence de sassafras permettant ainsi l'obtention d'un mélange isovanilline-vanilline qui sont deux isomères difficiles à séparer ;
- à partir d'eugénol, composé présent dans l'huile de clou de girofle, ce composé subissant une première étape d'isomérisation en isoeugénol (cis et trans en proportions variables) puis une deuxième étape d'oxydation pour donner la vanilline ; cependant, ce procédé chimique donne des rendements très moyens.

De plus, tous les procédés de l'art antérieur faisant appel à au moins une manipulation chimique, le produit obtenu ne peut qu'être appelé "vanilline de synthèse ou d'hémisynthèse" et ne peut en aucun cas être appelé "vanilline d'origine naturelle", même si le substrat de départ est naturel (cas de l'eugénol ou du safrole).

En ce qui concerne le benzaldéhyde, il est produit par exemple à partir d'aldéhyde cinnamique traité en milieu alcalin sous pression et à haute température (brevet US 4 617 419).

Quant à l'aldéhyde anisique, il est classiquement produit par oxydation chimique de l'anéthole que l'on trouve en grande quantité dans l'essence de badiane.

Mais, tout comme la vanilline et pour les mêmes raisons, ces deux composés ne peuvent pas être qualifiés de "naturels".

JP-A-2 200 192 décrit un procédé naturel de préparation d'aldéhydes consistant à mettre en contact une enzyme produite par une souche appartenant au genre Pseudomonas et capable de produire une enzyme à activité dioxygénase avec des dérivés styrèniques ayant une insaturation située exclusivement en a du noyau benzénique. L'activité de cette enzyme est faible. La nature pathogène-opportuniste du microorganisme qui la produit, constitue un obstacle à l'utilisation des aldéhydes obtenus dans des produits alimentaires, pharmaceutiques ou cosmétiques.

L'invention a pour but de remédier aux inconvénients de l'art antérieur et propose un procédé biotechnologique permettant la préparation de certaines substances aromatiques de bonne pureté avec des rendements élevés.

EP-A-0542348 (QUEST) décrit un procédé biotechnologique de préparation de substances aromatiques telles que la vanilline par action d'une lipoxygénase sur un substrat phénolique ayant une double liaison en a du noyau benzénique. Ce brevet européen fait partie de l'art antérieur selon l'article 54(3) CBE et ne désigne pas les états contractants LU et MC.

La Société demanderesse a eu le mérite d'établir qu'il était possible de préparer des substances aromatiques de formule : 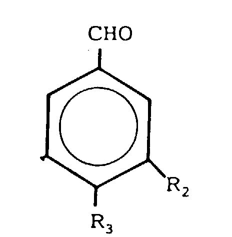 en soumettant l'un de leurs précurseurs, appelé substrat, choisi parmi l'eugénol, l'estragol, le chavicol, le safrole et les composés répondant à la formule : 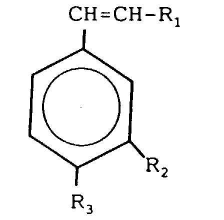 dans laquelle
R₁ peut être un radical -CH₃, -CH₂OH, -CHO, -COOH, 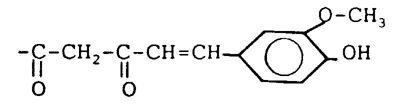
R₂ peut être un radical -H, -OH, -O-CH₃,
R₃ peut être un radical -H, -OH, -O-CH₃,
R₂ et R₃ peuvent former un pont méthylènedioxy 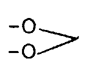 à l'action d'une enzyme : la lipoxygénase ou lipoxidase.

Cette enzyme est répertoriée sous la référence EC 1 13 11 12 dans l'ouvrage "Enzyme Nomenclature" (IU BMB Academic Press 1992).

Elle est est naturellement présente dans de nombreux végétaux tels que par exemple le soja, le blé ou la betterave, dans certains organes d'animaux tels que par exemple les foies de porc, de veau ou de poisson. Ces matières végétales ou animales peuvent donc constituer des sources industrielles de cette enzyme qui, de plus, peut également être synthétisée par certains microorganismes.

La lipoxygénase est connue notamment pour sa participation à la première étape d'un procédé permettant la synthèse d'aldéhydes à partir d'acides gras. En effet, son activité catalytique oxydative sur les acides gras insaturés, et plus particulièrement sur les acides linoléique et linolénique, donne lieu à la formation d'un hydroperoxyde pouvant ensuite être scindé en un oxoacide et un aldéhyde par une hydroperoxyde lyase.

Or, dans le cadre de l'invention, il a été trouvé que la lipoxygénase, quelle que soit son origine, pouvait à elle seule permettre la préparation de substances aromatiques de formule : 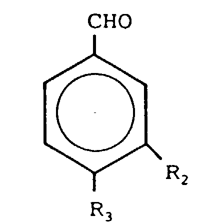 à partir de substrats choisis parmi l'eugénol, l'estragol, le chavicol et le safrole et les composés représentés par la formule ci-dessus mentionnée et selon les réactions suivantes : 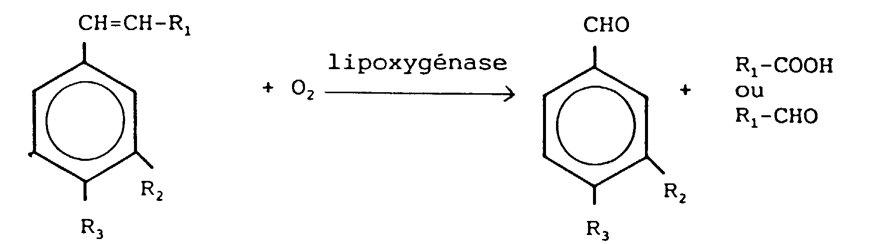 (R₁, R₂ et R₃ ayant les significations données précédemment).

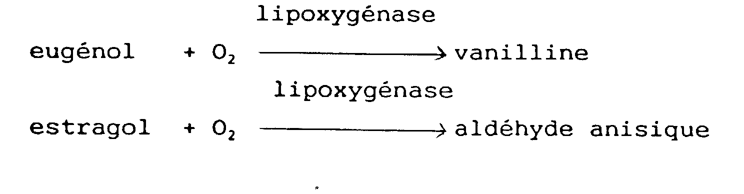

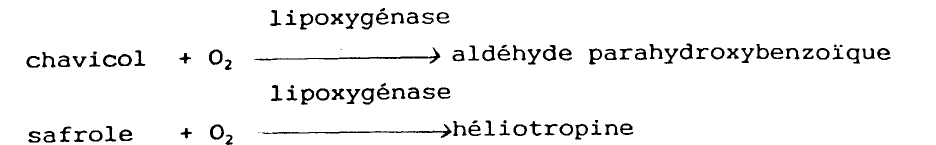

Les substances aromatiques préférées préparées selon le procédé conforme à l'invention sont récapitulées dans le tableau I.

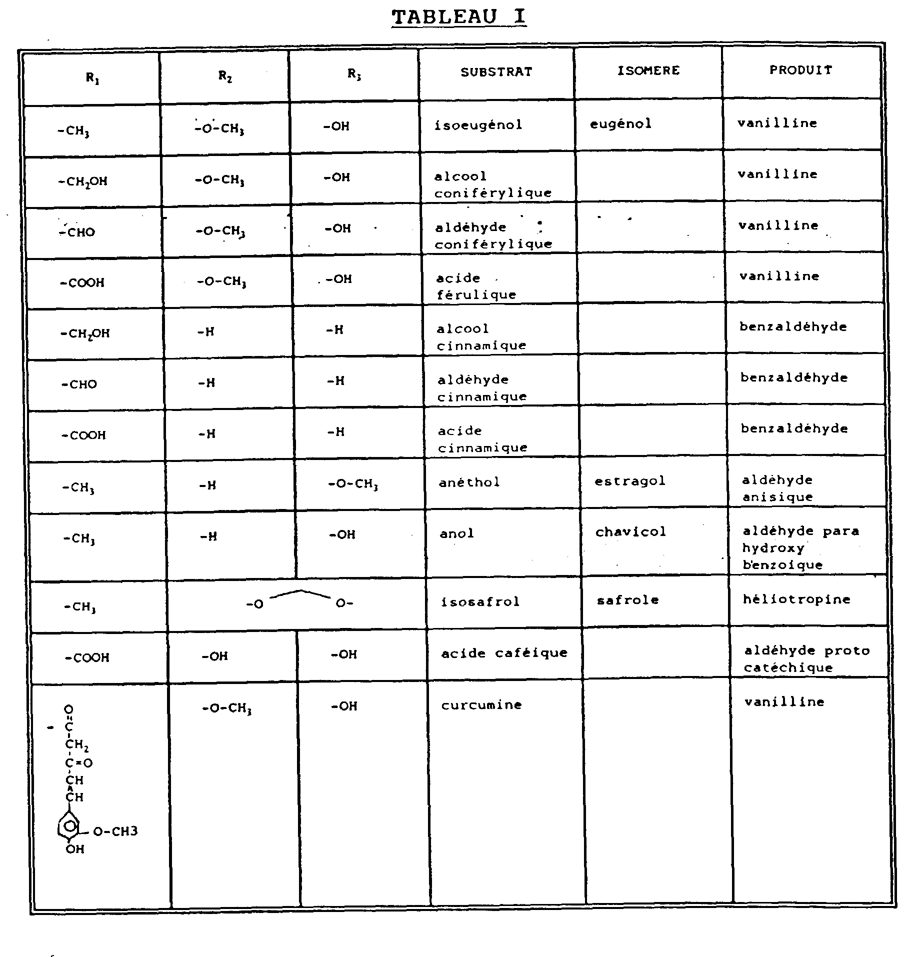

L'eugénol, le chavicol, l'estragol et le safrole qui sont respectivement les isomères de l'isoeugénol, de l'anol, de l'anéthole et de l'isosafrole peuvent eux-mêmes être transformés par la lipoxygénase en l'aldéhyde correspondant, soit la vanilline, l'aldéhyde hydroxybenzoïque, l'aldéhyde anisique et l'héliotropine mais avec un faible rendement plus faible.

En effet la condition la plus importante pour que la lipoxygénase puisse exercer son action est que le substrat comporte une liaison insaturée correctement positionnée : c'est le cas notamment de toutes les molécules représentées par la formule répondant au "substrat" dont la liaison insaturée située sur la chaîne portant R₁ ou CH₂ est positionnée soit en alpha, soit en bêta du cycle aromatique. C'est aussi le cas des exemples connus que sont les acides linoléique et linolénique qui voient soit leur carbone 9, soit leur carbone 13 (constituant un système cis-cis diénique non conjugé) être le siège d'une peroxydation.

Le procédé conforme à l'invention consiste donc à mettre en présence, en milieu tamponné, agité et thermo-régulé, une lipoxygénase et un substrat choisi parmi ceux mentionnés ci-dessus pendant un temps suffisant pour permettre la préparation de la substance aromatique correspondante, celle-ci pouvant être ensuite récupérée par des techniques classiques d'extraction par solvant ou par distillation.

Dans le procédé conforme à l'invention, la lipoxygénase peut être utilisée sous forme purifiée ou non purifiée.

Avantageusement :
- lorsqu'elle est purifiée c'est-à-dire généralement quand elle présente une activité supérieure à 30000 u/mg, (1 unité correspondant à une augmentation de l'absorbance à 234nm d'une valeur de 0,001 par minute lorsque l'acide linoléique est le substrat, à pH 9 et à 25°C), la lipoxygénase est utilisée à raison de 1 à 500 mg par gramme de substrat, de préférence de 5 à 100 mg par gramme de substrat et plus préférentiellement encore de 10 à 50 mg par gramme de substrat, (à titre d'exemple, on peut utiliser une lipoxygénase commercialisée par SIGMA sous forme d'une préparation enzymatique contenant au moins 60% de protéines), et
- lorsqu'elle n'est pas purifiée, c'est-à-dire lorsqu'elle contient moins de 7500 u/mg, comme c'est le cas dans les poudres acétoniques du commerce (forme préconcentrée à partir d'une matière première), ou dans un végétal frais broyé ou dans une poudre de farine végétale, ou encore, dans un organe animal frais broyé tel que le foie, elle sera utilisée à raison de 0,025 à 10 g par gramme de substrat et de préférence de 0,1 à 5 g par gramme de substrat. Lorsque le substrat mis en oeuvre est un composé ayant la formule représentée ci-dessus dans laquelle R₁ représente -CH₃, -CH₂OH ou -COOH et R₃ ne représente pas -OH, la lipoxygénase sous forme non purifiée peut être utilisée à raison de 0,01 à 50g par gramme de substrat.

Selon le procédé conforme à l'invention, les lipoxygénases préférentiellement utilisées sont la lipoxygénase de germes de soja et la lipoxygénase de germes de blé.

Selon un mode de réalisation avantageux du procédé conforme à l'invention, on établit la concentration en substrat de la lipoxygénase entre 2,5 et 100 g par litre de milieu de réaction et de préférence entre 20 et 75 g par litre de milieu de réaction.

Conformément à l'invention, ce milieu de réaction est un milieu aqueux tamponné. Les tampons utilisés sont des tampons classiques tels que le tampon phosphate de potassium et/ou le tampon phosphate de sodium et/ou le tampon borate de sodium et/ou le tampon phosphate-citrate-borate de sodium.

Avantageusement, les molarités de ces tampons sont comprises entre 0,05 M et 0,5 M et leur pH est compris entre 5 et 10. Lorsque le substrat mis en oeuvre est un composé ayant la formule représentée ci-dessus dans laquelle R₁ représente -CH₃, -CH₂OH ou -COOH et R₃ ne représente pas -OH, la plage de pH peut être étendue, le pH peut être compris entre 2 et 10 et est de préférence compris entre 5 et 10. Les ajustements de pH se font soit avec de la potasse, soit avec de la soude, soit avec de l'acide phosphorique.

Selon d'autres caractéristiques avantageuses de l'invention :
- la température du milieu de réaction varie entre 4 et 40°C et de préférence entre 22 et 32°C ;
- la durée de la réaction varie entre 30 minutes et 7 jours, de préférence entre 2 heures et 5 jours et est déterminée par suivi cinétique des réactions ;
- l'agitation doit être suffisamment forte, pour homogénéiser ou émulsionner l'ensemble du milieu réactionnel et pour oxygéner tout le substrat présent, et est réalisée à une vitesse de 150 tr/min.

Lorsque le substrat mis en oeuvre est un composé ayant la formule représentée ci-dessus dans laquelle R₁ représente -CH₃, -CH₂OH ou -COOH et R₃ ne représente pas -OH, la vitesse d'agitation peut être comprise entre 150 et 250 tr/min.

En outre, un agent émulsifiant tel que le TWEEN 80® peut être introduit dans le milieu à raison de 0,05 à 2% en volume par rapport au milieu de réaction.

Selon un mode de réalisation avantageux du procédé conforme à l'invention, en fonction du substrat utilisé, et en particulier quand le substrat est un composé de nature phénolique, du fait de la toxicité des phénols vis-à-vis de l'enzyme, il peut être nécessaire d'ajouter dans le milieu de réaction au moins un adsorbant choisi dans le groupe comprenant le charbon actif à une concentration comprise entre 5 et 50 g par litre, celui connu sous la marque Amberlite® de type XAD₂ à une concentration comprise entre 5 et 50 g par litre, la béta cyclodextrine à une concentration comprise entre 3 et 18 g par litre, de façon à protéger ladite enzyme. Lorsque le substrat mis en oeuvre est un composé de nature phénolique ayant la formule représentée ci-dessus dans laquelle R₁ représente -CH₃, -CH₂OH ou -COOH et R₃ ne représente pas -OH, on peut ajouter à la place ou en plus de l'adsorbant un complexant par exemple la polyvinylpolypyrrolidone (PVPP) à une concentration de 10 à 60 g par litre.

L'invention ne se limite pas strictement au procédé décrit, elle en embrasse au contraire toutes les variantes. La Société demanderesse a par exemple expérimenté de façon concluante la transformation du bêta-carotène en béta-ionone par l'action de la lipoxygénase qui agit dans ce cas au niveau d'une liaison insaturée située en position gamma par rapport au cycle de la molécule, conjuguée avec une liaison insaturée située en position alpha par rapport au même cycle.

Les exemples qui suivent permettront de mieux comprendre l'invention ; ils ne sont pas limitatifs et concernent les modes de réalisation avantageux de l'invention.

### EXEMPLE 1

Dans un flacon contenant 20 ml de tampon borate de sodium présentant une molarité de 0,1M et un pH de 6,5, on ajoute 0,75 g d'isoeugénol, 20 mg de lipoxygénase issue de germes de soja et titrant 36000 u/mg et 0,05 g de TWEEN 80®.

Après trois jours d'incubation à 28°C et sous agitation à une vitesse de 150 tr/min, l'ensemble de l'échantillon est extrait par du dichloroéthane et la vanilline est ensuite dosée par HPLC.

La production de vanilline est de 2,85 g/l.

### EXEMPLE 2

Dans les mêmes conditions que dans l'exemple 1, on ajoute au milieu de réaction 0,75 g d'eugénol à la place de l'isoeugénol en présence de 0,5 g de charbon actif.

Le rendement en vanilline est de 28,5 mg/litre.

### EXEMPLE 3

Huit flacons contenant les mêmes ingrédients que dans l'exemple 1 sont incubés à 28°C sur une table agitée à 150 tr/min. Chaque jour un échantillon est extrait par le dichloroéthane et dosé par HPLC.

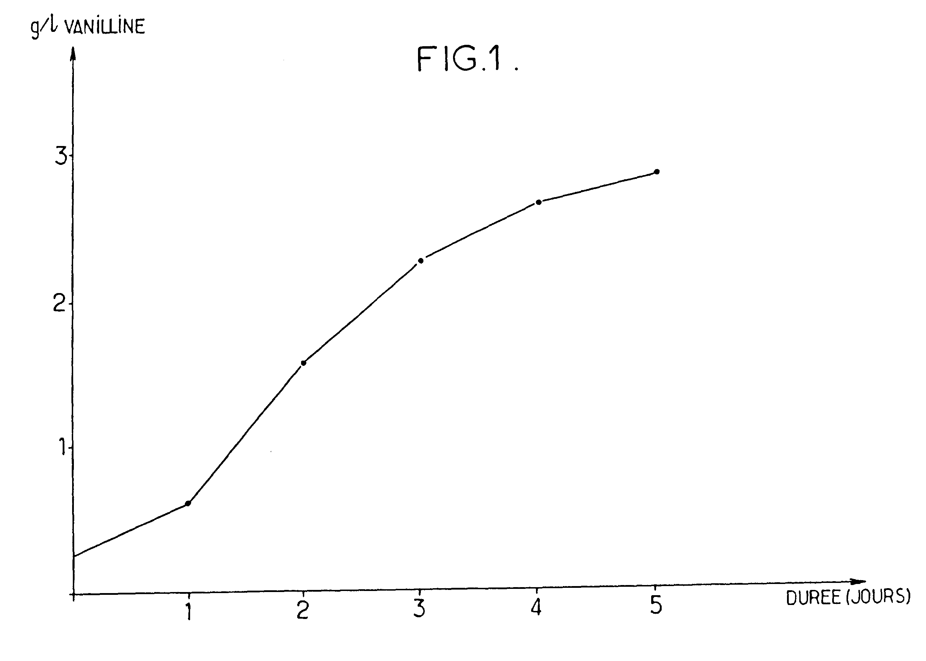

La cinétique est reproduite sur la figure 1.

La production de vanilline atteint 2,82 g/l.

### EXEMPLE 4

Dans les mêmes conditions que dans les exemples précédents, on fait varier les concentrations en isoeugénol de 5 à 100 g/l de milieu de réaction.

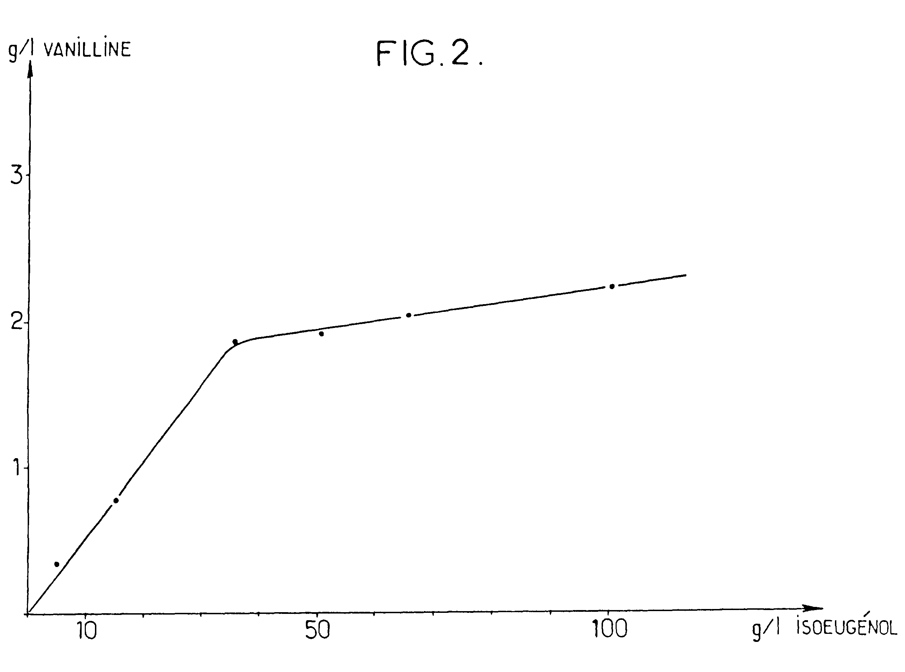

On obtient une production de vanilline, représentée sur la figure 2, atteignant 2,2 g/l.

### EXEMPLE 5

Dans les conditions décrites dans l'exemple 1, on fait varier la concentration de la lipoxygénase ajoutée au milieu de réaction de 0 à 2,5 g/l de milieu de réaction.

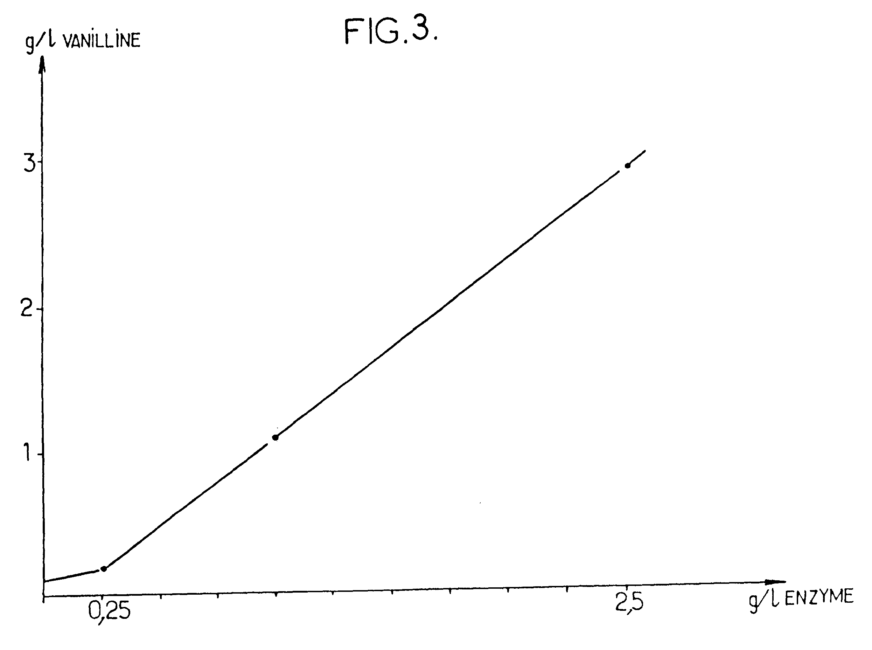

Les quantités de vanilline produites correspondantes sont représentées sur la figure 3.

La production de vanilline atteint 2,9 g/l.

### EXEMPLE 6

Dans les mêmes conditions que dans l'exemple 1, on ajoute 10 g/l de curcumine, préalablement traitée par de la potasse pour la solubiliser dans le milieu tampon.

La vanilline est obtenue avec un rendement de 37 mg/l.

### EXEMPLE 7

Dans les mêmes conditions que dans l'exemple 1, on remplace l'isoeugénol par 2 g/l d'alcool coniférylique.

En deux jours, la vanilline est obtenue avec un rendement de 240 mg/l.

### EXEMPLE 8

Dans les mêmes conditions que dans l'exemple 1, on ajoute 25 g/l d'aldéhyde cinnamique.

Après quatre jours d'incubation, on détecte 1,1 g/l de benzaldéhyde dans le milieu.

### EXEMPLE 9

Dans les mêmes conditions que dans l'exemple 1, on ajoute 8 g/l d'anéthole.

Après quatre jours, on mesure 448 mg/l d'aldéhyde anisique.

### EXEMPLE 10

Dans un flacon contenant 25 g d'aubergine broyée et 25 g de tampon borate de sodium présentant une molarité de 0,1 M et un pH de 6,5, on ajoute 0,4 g d'isoeugénol et 0,05g de TWEEN 80®. Après 3 jours d'incubation à 28°C et sous agitation à une vitesse de 200 tr/min, on détecte 1,75 g/l de vanilline.

### EXEMPLE 11

Dans un flacon contenant 20 g de tampon borate de sodium présentant une molarité de 0,1 M et un pH de 6,5 et 0,75 g d'isoeugénol, on ajoute 5 g de foie de porc frais broyé et 0,05 g de TWEEN 80®. Après 2 jours d'incubation à 28° C et sous agitation à une vitesse de 200 tr/min, la quantité de vanilline mesurée est de 0,7 g/l.

## Revendications (Revendications pour l'(les) Etat(s) contractant(s) suivant(s): AT, BE, CH, DE, DK, ES, FR, GB, GR, IE, IT, LI, NL, PT, SE)

1. Procédé de préparation de substances aromatiques de formule 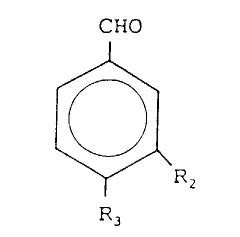 caractérisé par le fait que l'on soumet à l'action d'une lipoxygénase un substrat choisi parmi l'estragol, le safrole et les composés répondant à la formule : 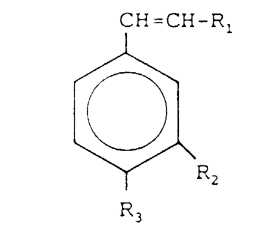 dans laquelle :
R₁ représente un radical -CH₃, -CH₂OH, -CHO, -COOH, 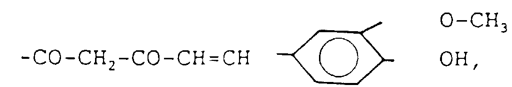
R₂ peut être un radical -H, -OH, -O-CH₃,
R₃ peut être un radical -H, -OH, -O-CH₃,
R₂ et R₃ peuvent former un pont méthylènedioxy 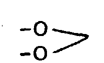 à la condition que lorsque R₁ représente -CH₃, CH₂OH ou -COOH, alors R₃ ne représente pas -OH, et lorsque R₁ représente 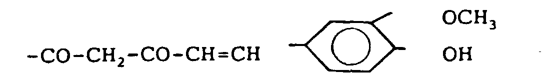 et R₂ représente OCH₃ alors R₃ ne représente pas -OH-.

2. Procédé selon la revendication 1, caractérisé par le fait que les substances aromatiques appartiennent au groupe comprenant: la vanilline, le benzaldéhyde, l'aldéhyde anisique, l'aldéhyde parahydroxybenzoïque, l'héliotropine et l'aldéhyde protocatéchique.

3. Procédé selon la revendication 1 ou 2, caractérisé par le fait que la lipoxygénase est utilisée sous forme purifiée à raison de 1 à 500 mg par gramme de substrat, de préférence entre 5 et 100 mg par gramme de substrat et plus préférentiellement encore de 10 à 50 mg par gramme de substrat.

4. Procédé selon la revendication 1 ou 2, caractérisé par le fait que la lipoxygénase est utilisée sous forme non purifiée à raison de 0,025 à 10 g par gramme de substrat et de préférence à raison de 0,1 à 5 g par gramme de substrat.

5. Procédé selon l'une des revendications 1 à 4, caractérisé par le fait que l'on utilise une lipoxygénase ou une source de lipoxygénase d'origine végétale.

6. Procédé selon l'une des revendications 1 à 4, caractérisé par le fait que l'on utilise une lipoxygénase ou une source de lipoxygénase d'origine animale.

7. Procédé selon l'une des revendications 1 à 6, caractérisé par le fait qu'on établit la concentration en substrat du milieu réactionnel entre 2,5 et 100 g par litre et de préférence entre 20 et 75 g par litre.

8. Procédé selon l'une des revendications 1 à 7, caractérisé par le fait que l'on tamponne le pH du milieu réactionnel entre 5 et 10.

9. Procédé selon l'une des revendications 1 à 8, caractérisé par le fait que l'on établit la température du milieu réactionnel entre 4 et 40°C et de préférence entre 22 et 32°C.

10. Procédé selon l'une des revendications 1 à 9, caractérisé par le fait que la durée de la réaction varie de 30 minutes à 7 jours et de préférence de 2 heures à 5 jours.

11. Procédé selon l'une des revendications 1 à 10, caractérisé par le fait que le milieu réactionnel est agité à une vitesse de 150 tr/min.

12. Procédé selon l'une des revendications 1 à 11, caractérisé par le fait que l'on ajoute dans le milieu réactionnel un agent émulsifiant à raison de 0,05 à 2% en volume.

13. Procédé selon l'une des revendications 1 à 12, caractérisé par le fait que, dans le cas où le substrat est un composé de nature phénolique, on ajoute au moins un adsorbant dans le milieu réactionnel.

14. Procédé selon la revendication 1 ou 2, caractérisé par le fait que la lipoxygénase est utilisée sous forme non purifiée à raison de 0,01 à 50 g par gramme de substrat.

15. Procédé selon l'une des revendications 1 à 7, caractérisé par le fait que l'on tamponne le pH du milieu réactionnel entre 2 et 5.

16. Procédé selon l'une des revendications 1 à 10, caractérisé par le fait que le milieu réactionnel est agité à une vitesse comprise entre 150 et 250 tr/min.

17. Procédé selon l'une des revendications 1 à 12, caractérisé par le fait que, dans le cas où le substrat est un composé de nature phénolique, on ajoute au moins un complexant dans le milieu réactionnel.

## Revendications (Revendications pour l'(les) Etat(s) contractant(s) suivant(s): LU, MC)

1. Procédé de préparation de substances aromatiques de formule 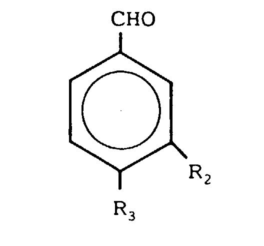 caractérisé par le fait que l'on soumet à l'action d'une lipoxygénase un substrat choisi parmi l'eugénol, l'estragol, le chavicol, le safrole et les composés répondant à la formule : 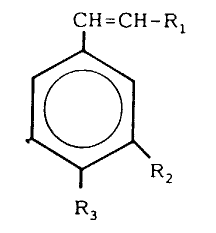 dans laquelle :
R₁ représente un radical -CH₃, -CH₂OH, -CHO, -COOH, 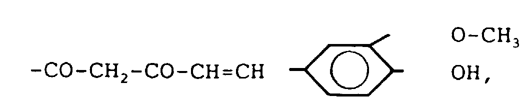
R₂ peut être un radical -H, -OH, -O-CH₃,
R₃ peut être un radical -H, -OH, -O-CH₃,
R₂ et R₃ peuvent former un pont méthylènedioxy

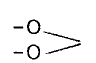

2. Procédé selon la revendication 1, caractérisé par le fait que les substances aromatiques appartiennent au groupe comprenant: la vanilline, le benzaldéhyde, l'aldéhyde anisique, l'aldéhyde parahydroxybenzoïque, l'héliotropine et l'aldéhyde protocatéchique.

3. Procédé selon la revendication 1 ou 2, caractérisé par le fait que la lipoxygénase est utilisée sous forme purifiée à raison de 1 à 500 mg par gramme de substrat, de préférence entre 5 et 100 mg par gramme de substrat et plus préférentiellement encore de 10 à 50 mg par gramme de substrat.

4. Procédé selon la revendication 1 ou 2, caractérisé par le fait que la lipoxygénase est utilisée sous forme non purifiée à raison de 0,025 à 10 g par gramme de substrat, de préférence à raison de 0,1 à 5 g par gramme de substrat.

5. Procédé selon l'une des revendications 1 à 4, caractérisé par le fait que l'on utilise une lipoxygénase ou une source de lipoxygénase d'origine végétale.

6. Procédé selon l'une des revendications 1 à 4, caractérisé par le fait que l'on utilise une lipoxygénase ou une source de lipoxygénase d'origine animale.

7. Procédé selon l'une des revendications 1 à 6, caractérisé par le fait qu'on établit la concentration en substrat de la lipoxygénase entre 2,5 et 100 g par litre de milieu de réaction et de préférence entre 20 et 75 g par litre de milieu de réaction.

8. Procédé selon l'une des revendications 1 à 7, caractérisé par le fait qu'il est mis en oeuvre dans un milieu tamponné présentant un pH compris entre 5 et 10.

9. Procédé selon l'une des revendications 1 à 8, caractérisé par le fait que la température du milieu de réaction est comprise entre 4 et 40°C et de préférence entre 22 et 32°C.

10. Procédé selon l'une des revendications 1 à 9, caractérisé par le fait que la durée de la réaction varie de 30 minutes à 7 jours et de préférence de 2 heures à 5 jours.

11. Procédé selon l'une des revendications 1 à 10, caractérisé en ce qu'il est mis en oeuvre sous agitation à une vitesse de 150 tr/min.

12. Procédé selon l'une des revendications 1 à 11, caractérisé par le fait qu'un agent émulsifiant est ajouté à raison de 0,05 à 2% en volume par rapport au milieu de réaction.

13. Procédé selon l'une des revendications 1 à 11, caractérisé par le fait que l'on peut ajouter dans le milieu de réaction, dans le cas où un composé de nature phénolique est utilisé comme substrat, au moins un adsorbant.

14. Procédé selon la revendication 13, caractérisé par le fait que l'adsorbant est choisi parmi le groupe comprenant le charbon actif à une concentration comprise entre 5 et 50g par litre, celui connu sous la marque "Amberlite®" de type XAD₂ à une concentration comprise entre 5 et 50g par litre, la béta cyclodextrine à une concentration comprise entre 3 et 18 g par litre.

15. Procédé selon la revendication 1 ou 2, caractérisé par le fait que le substrat est tel que lorsque R₁ représente -CH₃, -CH₂OH ou -COOH, R₃ ne représente pas -OH et que la lipoxygénase sous forme non purifiée est utilisée à raison de 0,01 à 50g par gramme de substrat.

16. Procédé selon l'une quelconque des revendications 1 à 7, caractérisé par le fait que le substrat est tel que lorsque R₁ représente -CH₃, -CH₂OH ou -COOH, R₃ ne représente pas -OH et que l'on tamponne le pH du milieu réactionnel entre 2 et 5.

17. Procédé selon l'une des revendications 1 à 10, caractérisé par le fait que le substrat est tel que lorsque R₁ représente -CH₃, -CH₂OH ou -COOH, R₃ ne représente pas -OH et que le milieu réactionnel est agité à une vitesse comprise entre 150 et 250 tr/min.

18. Procédé selon l'une des revendications 1 à 12, caractérisé par le fait que le substrat est tel que lorsque R₁ représente -CH₃, -CH₂OH ou -COOH, R₃ ne représente pas -OH et que, dans le cas où le substrat est un composé de nature phénolique, on ajoute au moins un complexant dans le milieu réactionnel, par exemple la polyvinyl polypyrrolidone (PVPP) à une concentration comprise entre 10 et 60 g/l.

## Patentansprüche (Patentansprüche für folgende(n) Vertragsstaat(en): AT, BE, CH, DE, DK, ES, FR, GB, GR, IE, IT, LI, NL, PT, SE)

1. Verfahren zur Herstellung von aromatischen Substanzen mit der Formel 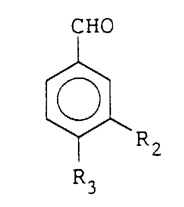 dadurch gekennzeichnet, daß der Einwirkung einer Lipoxygenase ein Substrat unterzogen wird, welches ausgewählt ist unter Estragol, Safrol sowie Zusammensetzungen, die der Formel 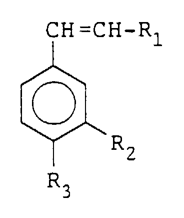 entsprechen, in welcher:
R₁ für einen Rest -CH₃, -CH₂OH, -CHO, -COOH, 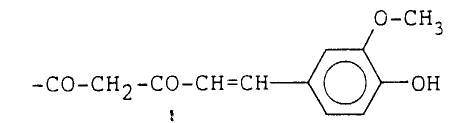 steht,
R₂ ein Rest -H, -OH, -O-CH₃ sein kann,
R₃ ein Rest -H, -OH, -O-CH₃ sein kann,
R₂ und R₃ eine Methylendioxybrücke 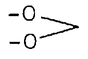 bilden können,
mit der Bedingung, daß, falls R₁ für -CH₃, CH₂OH oder -COOH steht, R₃ in diesem Fall nicht für -OH steht, und falls R₁ für 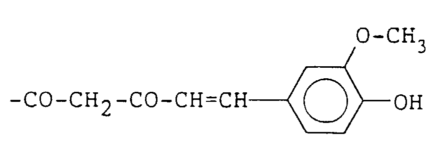 steht und R₂ für OCH₃ steht, R₃ in diesem Fall nicht für -OH- steht.

2. Verfahren nach Anspruch 1, dadurch gekennzeichnet, daß die aromatischen Substanzen der Gruppe angehören, welche aufweist: Vanillin, Benzaldehyd, Anisaldehyd, Parahydroxybenzaldehyd, Heliotropin und Protocatechualdehyd.

3. Verfahren nach Anspruch 1 oder 2, dadurch gekennzeichnet, daß Lipoxygenase in gereinigter Form in einem Verhältnis von 1 bis 500 mg pro Gramm Substrat, bevorzugt zwischen 5 und 100 mg pro Gramm Substrat, und insbesondere bevorzugt zwischen 10 und 50 mg pro Gramm Substrat eingesetzt wird.

4. Verfahren nach Anspruch 1 oder 2, dadurch gekennzeichnet, daß Lipoxygenase in nicht-gereinigter Form in einem Verhältnis von 0,025 bis 10 g pro Gramm Substrat und bevorzugt in einem Verhältnis von 0,1 bis 5 g pro Gramm Substrat eingesetzt wird.

5. Verfahren nach einem der Ansprüche 1 bis 4, dadurch gekennzeichnet, daß eine Lipoxygenase bzw. eine Lipoxygenasequelle eingesetzt wird, die pflanzlichen Ursprungs ist.

6. Verfahren nach einem der Ansprüche 1 bis 4, dadurch gekennzeichnet, daß eine Lipoxygenase bzw. eine Lipoxygenasequelle eingesetzt wird, die tierischen Ursprungs ist.

7. Verfahren nach einem der Ansprüche 1 bis 6, dadurch gekennzeichnet, daß die Substratkonzentration des Reaktionsmediums auf zwischen 2,5 und 100 g pro Liter und bevorzugt auf zwischen 20 und 75 g pro Liter eingestellt wird.

8. Verfahren nach einem der Ansprüche 1 bis 7, dadurch gekennzeichnet, daß der pH des Reaktionsmediums auf zwischen 5 und 10 gepuffert wird.

9. Verfahren nach einem der Ansprüche 1 bis 8, dadurch gekennzeichnet, daß die Temperatur des Reaktionsmediums auf zwischen 4 und 40°C und bevorzugt auf zwischen 22 und 32°C eingestellt wird.

10. Verfahren nach einem der Ansprüche 1 bis 9, dadurch gekennzeichnet, daß die Reaktionsdauer von 30 Minuten bis 7 Tage und bevorzugt von 2 Stunden bis 5 Tage variiert.

11. Verfahren nach einem der Ansprüche 1 bis 10, dadurch gekennzeichnet, daß das Reaktionsmedium mit einer Geschwindigkeit von 150 U/min. gerührt wird.

12. Verfahren nach einem der Ansprüche 1 bis 11, dadurch gekennzeichnet, daß dem Reaktionsmedium ein Emulgator in einem Verhältnis von 0,05 bis 2 Vol.-% zugegeben wird.

13. Verfahren nach einem der Ansprüche 1 bis 12, dadurch gekennzeichnet, daß, falls das Substrat eine phenolische Verbindung ist, dem Reaktionsmedium mindestens ein Adsorptionsmittel zugegeben wird.

14. Verfahren nach Anspruch 1 oder 2, dadurch gekennzeichnet, daß Lipoxygenase in nicht-gereinigter Form in einem Verhältnis von 0,01 bis 50 g pro Gramm Substrat eingesetzt wird.

15. Verfahren nach einem der Ansprüche 1 bis 7, dadurch gekennzeichnet, daß der pH des Reaktionsmediums auf zwischen 2 und 5 gepuffert wird.

16. Verfahren nach einem der Ansprüche 1 bis 10, dadurch gekennzeichnet, daß das Reaktionsmedium mit einer zwischen 150 und 250 U/min. liegenden Geschwindigkeit gerührt wird.

17. Verfahren nach einem der Ansprüche 1 bis 12, dadurch gekennzeichnet, daß, falls das Substrat eine phenolische Verbindung ist, dem Reaktionsmedium zumindest ein Komplexbildner zugegeben wird.

## Patentansprüche (Patentansprüche für folgende(n) Vertragsstaat(en): LU, MC)

1. Verfahren zur Herstellung von aromatischen Substanzen mit der Formel 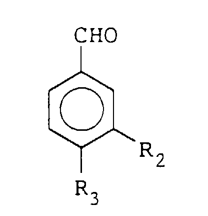 dadurch gekennzeichnet, daß der Einwirkung einer Lipoxygenase ein Substrat unterzogen wird, welches ausgewählt ist unter Eugenol, Estragol, Chavicol, Safrol sowie Zusammensetzungen, die der Formel 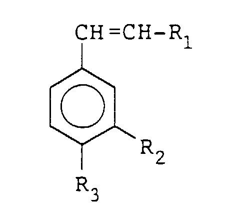 entsprechen, in welcher:
R₁ für einen Rest -CH₃, -CH₇OH, -CHO, -COOH, 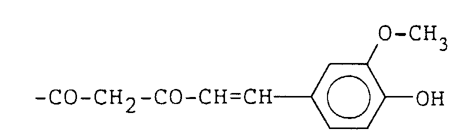 steht,
R₂ ein Rest -H, -OH, -O-CH₃ sein kann,
R₃ ein Rest -H, -OH, -O-CH₃ sein kann,
R₂ und R₃ eine Methylendioxybrücke 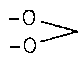 bilden können.

2. Verfahren nach Anspruch 1, dadurch gekennzeichnet, daß die aromatischen Substanzen der Gruppe angehören, welche aufweist: Vanillin, Benzaldehyd, Anisaldehyd, Parahydroxybenzaldehyd, Heliotropin und Protocatechualdehyd.

3. Verfahren nach Anspruch 1 oder 2, dadurch gekennzeichnet, daß Lipoxygenase in gereinigter Form in einem Verhältnis von 1 bis 500 mg pro Gramm Substrat, bevorzugt zwischen 5 und 100 mg pro Gramm Substrat, und insbesondere bevorzugt zwischen 10 und 50 mg pro Gramm Substrat eingesetzt wird.

4. Verfahren nach Anspruch 1 oder 2, dadurch gekennzeichnet, daß Lipoxygenase in nicht-gereinigter Form in einem Verhältnis von 0,025 bis 10 g pro Gramm Substrat und bevorzugt in einem Verhältnis von 0,1 bis 5 g pro Gramm Substrat eingesetzt wird.

5. Verfahren nach einem der Ansprüche 1 bis 4, dadurch gekennzeichnet, daß eine Lipoxygenase bzw. eine Lipoxygenasequelle eingesetzt wird, die pflanzlichen Ursprungs ist.

6. Verfahren nach einem der Ansprüche 1 bis 4, dadurch gekennzeichnet, daß eine Lipoxygenase bzw. eine Lipoxygenasequelle eingesetzt wird, die tierischen Ursprungs ist.

7. Verfahren nach einem der Ansprüche 1 bis 6, dadurch gekennzeichnet, daß die Substratkonzentration von Lipoxygenase auf zwischen 2,5 und 100 g pro Liter Reaktionsmedium und bevorzugt auf zwischen 20 und 75 g pro Liter Reaktionsmedium eingestellt wird.

8. Verfahren nach einem der Ansprüche 1 bis 7, dadurch gekennzeichnet, daß es in einem gepufferten Medium durchgeführt wird, das einen pH-Wert zwischen 5 und 10 aufweist.

9. Verfahren nach einem der Ansprüche 1 bis 8, dadurch gekennzeichnet, daß die Temperatur des Reaktionsmediums zwischen 4 und 40°C und bevorzugt zwischen 22 und 32°C liegt.

10. Verfahren nach einem der Ansprüche 1 bis 9, dadurch gekennzeichnet, daß die Reaktionsdauer von 30 Minuten bis 7 Tage und bevorzugt von 2 Stunden bis 5 Tage variiert.

11. Verfahren nach einem der Ansprüche 1 bis 10, dadurch gekennzeichnet, daß es unter Rühren mit einer Geschwindigkeit von 150 U/min. durchgeführt wird.

12. Verfahren nach einem der Ansprüche 1 bis 11, dadurch gekennzeichnet, daß ein Emulgator in einem Verhältnis von 0,05 bis 2 Vol.-% in Bezug auf das Reaktionsmedium zugegeben wird.

13. Verfahren nach einem der Ansprüche 1 bis 11, dadurch gekennzeichnet, daß dem Reaktionsmedium, falls eine phenolische Verbindung als Substrat eingesetzt wird, mindestens ein Adsorptionsmittel zugegeben werden kann.

14. Verfahren nach Anspruch 13, dadurch gekennzeichnet, daß das Adsorptionsmittel aus der Gruppe ausgewählt ist, die aufweist: Aktivkohle mit einer Konzentration von zwischen 5 und 50 g pro Liter, das unter der Markenbezeichnung "Amberlite®" bekannte vom Typ XAD₂ mit einer Konzentration von zwischen 5 und 50 g pro Liter, β-Cyclodextrin mit einer Konzentration von zwischen 3 und 18 g pro Liter.

15. Verfahren nach Anspruch 1 oder 2, dadurch gekennzeichnet, daß das Substrat derart ist, daß, falls R₁ für -CH₃, CH₂OH oder -COOH steht, R₃ nicht für -OH steht, und daß Lipoxygenase in nicht-gereinigter Form in einem Verhältnis von 0,01 bis 50 g pro Gramm Substrat eingesetzt wird.

16. Verfahren nach einem der Ansprüche 1 bis 7, dadurch gekennzeichnet, daß das Substrat derart ist, daß, falls R₁ für -CH₃, CH₂OH oder -COOH steht, R₃ nicht für -OH steht, und daß der pH des Reaktionsmediums auf zwischen 2 und 5 gepuffert wird.

17. Verfahren nach einem der Ansprüche 1 bis 10, dadurch gekennzeichnet, daß das Substrat derart ist, daß, falls R₁ für -CH₃, CH₂OH oder -COOH steht, R₃ nicht für -OH steht, und daß das Reaktionsmedium mit einer zwischen 150 und 250 U/min. liegenden Geschwindigkeit gerührt wird.

18. Verfahren nach einem der Ansprüche 1 bis 12, dadurch gekennzeichnet, daß das Substrat derart ist, daß, falls R₁ für -CH₃, CH₂OH oder -COOH steht, R₃ nicht für -OH steht, und daß, falls das Substrat eine phenolische Verbindung ist, dem Reaktionsmedium zumindest ein Komplexbildner zugegeben wird, beispielsweise Polyvinylpyrrolidon (PVPP) mit einer Konzentration von zwischen 10 und 60 g/l.

## Claims (Claims for the following Contracting State(s): AT, BE, CH, DE, DK, ES, FR, GB, GR, IE, IT, LI, NL, PT, SE)

1. Method for the preparation of aromatic substances of formula 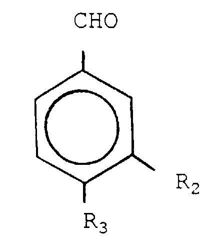 characterized by the fact that a substrate, chosen from estragol, safrole and compounds corresponding to the formula : 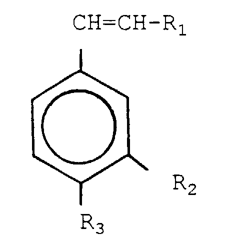 in which
R₁ represents a radical -CH₃, -CH₂OH, -CHO, -COOH, 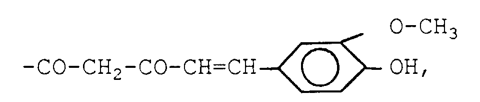
R₂ may be a radical -H, -OH, -O-CH₃,
R₃ may be a radical -H, -OH, -O-CH₃,
R₂ and R₃ may form a methylenedioxy bridge 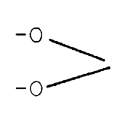 is subjected to the action of a lipoxygenase, provided that when R₁ represents -CH₃, CH₂OH, or -COOH, then
R₃ cannot represent -OH, and when R₁ represents 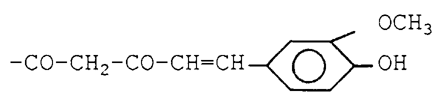 and R₂ represents OCH₃, then R₃ does not represent -OH-.

2. Method according to claim 1, characterized in that the aromatic substances belong to the group comprising : vanillin, benzaldehyde, anisaldehyde, parahydroxybenzaldehyde, heliotropin and protocatechic aldehyde.

3. Method according to claim 1 or claim 2, characterized in that the lipoxygenase is used in purified form in an amount of 1 to 500 mg per gram of substrate, preferably between 5 and 100 mg per gram of substrate and even more preferably from 10 to 50 mg per gram of substrate.

4. Method according to claim 1 or claim 2, characterized in that the lipoxygenase is used in unpurified form in an amount of 0.025 to 10 g per gram of substrate and preferably in an amount of 0.1 to 5 g per gram of substrate.

5. Method according to any one of claims 1 to 4, characterized in that a lipoxygenase or a source of lipoxygenase of plant origin is used.

6. Method according to any one of claims 1 to 4, characterized in that a lipoxygenase or a source of lipoxygenase of animal origin is used.

7. Method according to any one of claims 1 to 6, characterized in that the concentration of the substrate in the reaction medium is established between 2.5 and 100 g per litre, and preferably between 20 and 75 g per litre.

8. Method according to any one of claims 1 to 7, characterized in that the pH of the reaction medium is buffered between 5 and 10.

9. Method according to claims 1 to 8, characterized in that the temperature of the reaction medium is between 4 and 40°C and preferably between 22 and 32°C.

10. Method according to any one of claims 1 to 9, characterized in that the duration of the reaction varies from 30 minutes to 7 days, and preferably from 2 hours to 5 days.

11. Method according to any one of claims 1 to 10, characterized in that the reaction medium is stirred at a speed of 150 rev/min.

12. Method according to any one of claims 1 to 11, characterized in that an emulsifying agent is added in the reaction medium in an amount of 0.05 to 2 % by volume.

13. Method according to any one of claims 1 to 12, characterized in that at least one adsorbing agent is added in the reaction medium, in the case where a compound of phenolic nature is used as substrate.

14. Method according to claim 1 or claim 2, characterized in that the lipoxygenase is used in unpurified form in an amount of 0.01 to 50 g per gram of substrate.

15. Method according to any of claims 1 to 7, characterized in that the pH of the reaction medium is buffered between 2 and 5.

16. Method according to any one of claims 1 to 10, characterized in that the reaction medium is stirred at a speed comprised between 150 and 250 rev/min.

17. Method according to any one of claims 1 to 12, characterized in that at least one complexing agent is added in the reaction medium, in the case where a compound of phenolic nature is used as substrate.

## Claims (Claims for the following Contracting State(s): LU, MC)

1. Method for the preparation of aromatic substances of formula 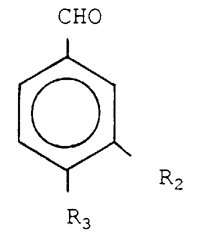 characterized in that a substrate, chosen from eugenol, estragol, chavicol or safrole and compounds corresponding to the formula : 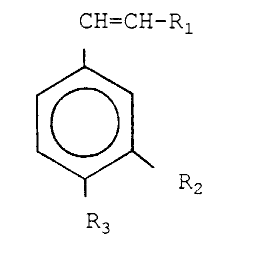 in which
R₁ represents a radical -CH₃, -CH₂OH, -CHO, -COOH, 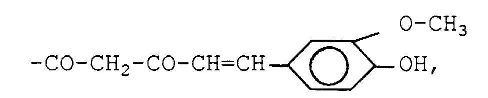
R₂ may be a radical -H, -OH, -O-CH₃,
R₃ may be a radical -H, -OH, -O-CH₃,
R₂ and R₃ may form a methylenedioxy bridge 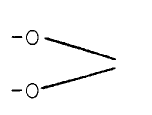 is subjected to the action of a lipoxygenase.

2. Method according to claim 1, characterized in that aromatic substances belong to the group comprising vanillin, benzaldehyde, anisaldehyde, parahydroxybenzaldehyde, heliotropin and protocatechic aldehyde.

3. Method according to claim 1 or claim 2, characterized in that the lipoxygenase is used in purified form in an amount of 1 to 500 mg per gram of substrate, preferably between 5 and 100 mg per gram of substrate and even more preferably from 10 to 50 mg per gram of substrate.

4. Method according to claim 1 or claim 2, characterized in that the lipoxygenase is used in unpurified form in an amount of 0.025 to 10 g per gram of substrate and preferably in an amount of 0.1 to 5 g per gram of substrate.

5. Method according to any one of claims 1 to 4, characterized in that a lipoxygenase or a source of lipoxygenase of plant origin is used.

6. Method according to any one of claims 1 to 4, characterized in that a lipoxygenase or a source of lipoxygenase of animal origin is used.

7. Method according to any one of claims 1 to 6, characterized in that the concentration of the substrate in the reaction medium is established between 2.5 and 100 g per litre, and preferably between 20 and 75 g per litre.

8. Method according to any one of claims 1 to 7, characterized in that the pH of the reaction medium is buffered between 5 and 10.

9. Method according to claims 1 to 8, characterized in that the temperature of the reaction medium is between 4 and 40°C and preferably between 22 and 32°C.

10. Method according to any one of claims 1 to 9, characterized in that the duration of the reaction varies from 30 minutes to 7 days, and preferably from 2 hours to 5 days.

11. Method according to any one of claims 1 to 10, characterized in that the reaction medium is stirred at a speed of 150 rev/min.

12. Method according to any one of claims 1 to 11, characterized in that an emulsifying agent is added in the reaction medium in an amount of 0.05 to 2 % by volume.

13. Method according to any one of claims 1 to 11, characterized in that at least one adsorbing agent is added in the reaction medium, in the case where a compound of phenolic nature is used as substrate.

14. Method according to claim 13, wherein the adsorbing agent is chosen among the group comprising activated charcoal at a concentration between 5 and 50 g per litre, the latter known under the trade name "Amberlite" of type XAD₂ at a concentration between 5 and 50 g per litre, betacyclodextrin at a concentration between 3 and 18 g per litre.

15. Method according to claim 1 or claim 2, wherein the substrate is so that when R₁ represents -CH₃, -CH₂OH, or -COOH, then R₃ cannot represent -OH and that lipoxygenase is used in unpurified form in an amount of 0.01 to 50 g per gram of substrate.

16. Method according to any of claims 1 to 7, wherein the substrate is so that when R₁ represents -CH₃, -CH₂OH, or -COOH, then R₃ cannot represent -OH and that the pH of the reaction medium is buffered between 2 and 5.

17. Method according to any one of claims 1 to 10, wherein the substrate is so that when R₁ represents -CH₃, -CH₂OH, or -COOH, then R₃ cannot represent -OH and that the reaction medium is stirred at a speed between 150 and 250 rev/min.

18. Method according to any one of claims 1 to 12, wherein the substrate is so that when R₁ represents -CH₃, -CH₂OH, or -COOH, then R₃ cannot represent -OH and that at least one complexing agent is added in the reaction medium, for example polyvinylpolypyrrolidone (PVPP) at a concentration between 10 and 60 g per litre in the case where a compound of phenolic nature is used as substrate.
